# EUROPEAN PATENT APPLICATION

(11) **EP 4 293 037 A1**
(43) Date of publication of application: **20.12.2023**
(21) Application number: 22752258.8
(22) Date of filing: 09.02.2022
(51) Int. Cl.: C07K 7/00, A61P 25/00

(54) **ARTIFICIAL SMALL MOLECULE INTERFERING PEPTIDE OF DAPK1 PHOSPHORYLATED SUBSTRATE AND PHARMACEUTICAL USE THEREOF**

(30) Priority: 10.02.2021 CN 202110183968; 29.01.2022 CN 202210110300
(71) Applicant: Beijing Sinancheng Technology co., Ltd., Beijing 100075 (CN)
(72) Inventor: CUI, Hong, Toronto, Ontario M2N7B8 (CA)
(74) Representative: Murgitroyd & Company
(86) International application number: PCT/CN2022/075581
(87) International publication number: WO 2022/171098

(57) **Abstract**

This invention" An artificial short interfering peptide for the phosphorylation substrate of DAPK1 and its potential pharmaceutical applications.", relates to the field of peptide drug development; the peptide can be used in preparation of drugs for prevention and treatment of ischemic stroke, as well as the drugs for the disease that based on the same mechanism, such as traumatic brain injury, spinal cord injury, neonatal hypoxic-ischemic encephalopathy, depression, autism and neurodegenerative diseases like multiple sclerosis, Alzheimer's disease, amyotrophic lateral sclerosis, Parkinson's disease, and Huntington's disease.

## Description

### Technical Field

The present invention relates to the field of short peptide drug development, particularly to an artificial short interfering peptide that targets the phosphorylation substrate of DAPK1, and its pharmaceutical applications.

### Background

Strokes are also known as cerebrovascular accidents (CVA), a disease that affects the arteries leading to focal neurological deficits. It is characterized by high morbidity, mortality, disability, recurrence, medical costs and complications, but awareness, treatment, and control rates are poor. According to the World Health Organization, stroke is the second leading cause of death worldwide and the first cause of disability in adults, seriously affecting human health and quality of life, as well as on their family, community, and economy. According to the "China Stroke Prevention Report 2018", stroke is the first cause of death and disability among adults in China, accounting for about 1/3 of deaths from cerebrovascular disease worldwide. 1.96 million people die from cerebrovascular disease each year, with 12.42 million surviving patients, and the incidence is rapidly increasing and trending younger.

Stroke can be caused either by a clot obstructing the flow of blood to the brain (called an **ischemic stroke)** or by a blood vessel rupturing and preventing blood flow to the brain (called a **hemorrhagic stroke),** of which ischemic stroke accounts for about 87%.It is a serious neurological disease caused by interruption of blood flow due to blood clot formation or embolism. It is a serious neurological disease caused by interruption of blood flow due to thrombosis or embolism. The current effective treatment is thrombolytic therapy with recombinant tissue fibrinogen activator (rtPA). The treatment is currently effective. However, only about 5% of patients can be treated with thrombolysis, and most patients can only be treated with symptomatic support due to the limited window for effective stroke treatment, the unpredictable length of the procedure, and the issues with blood reperfusion injury and intracranial haemorrhage. Because neuroprotective agents can be used in hemorrhagic stroke, their therapeutic effects and prospects are promising. They can be applied to protect normal nerve cells and salvage undead neural tissue, which can reduce the area of cerebral infarction and at the same time avoid the complications of thrombolytic or anticoagulant treatment regimens, without detailed etiologic differential diagnosis before adoption. so, their therapeutic effects and prospects are promising, and are a recent It is a hot spot for research. However, more than 1000 small molecule compounds have been developed by laboratories worldwide for neuroprotective agents in ischemic stroke, and more than 200 clinical trials have been conducted. At this point, there isn't a single neuroprotective medication that is Generally accepted, thus it's critical to keep researching and developing new therapies to reduce neuronal cell death and treat stroke effectively.

In ischemic stroke, neurons are depleted of cellular energy due to hypoxia and glucose deprivation, resulting in a complex cascade of responses involving multiple mechanisms. A complex cascade of reactions in which interactions lead to neuronal damage and death. These responses include imbalance of ion homeostasis, generation of reactive oxygen species and nitrogen species (ROS and RNS), and mitochondrial dysfunction, which ultimately leads to cell death through either necrosis or apoptosis. Among the cellular pathways involved in the cascade reaction, Death Associated Protein Kinase1 (DAPK1), a calmodulin-regulated serine/threonine protein kinase, plays an important role. It has been shown that the DAPK1-NR2B pathway, DAPK1-DANGER pathway, DAPK1-p53 pathway and DAPK1-Tau pathway are all involved in hypoxia and glucose-deprivation-induced cell death in animal ischemic-hypoxia. Blocking these cascades is effective in reducing neuronal death in the acute treatment of animal models of ischemia and hypoxia. but no effective drugs have been successfully developed for clinical treatment based on this, so there is a need to develop more drugs for ischemic stroke. There is a need for new medications related to DAPK1 for treat ischemic stroke.

Peptide, generally refers to a small molecule protein containing less than 100 amino acids, has the incomparable advantages of small molecule compound and protein and other large molecule drugs: like small molecule compound, it does not need to be digested, can be administered directly and absorbed quickly, almost 100% absorption and utilization without energy consumption, does not lead to gastrointestinal tract function burden and metabolic burden, can be absorbed as a carrier, and has the same molecular cognition as large molecule. It possesses great biological activity, considerable pharmacodynamics, clear conformational relationships, good molecular cognition, low toxicity, few to no side effects, and no accumulation. In recent years, it has become a key area for medication development.

### Summary

In view of the described needs in this field, the present invention focuses on the development of short peptide drugs for the treatment of ischemic stroke. According to research findings, a method of drugs development for preventing and treating ischemic stroke with a pharmaceutical agent is provided, as well as a short peptide and its intermediate products (such as gene sequences, expression vectors) for use in the pharmaceutical production for preventing and treating ischemic stroke.

Specifically, the present invention disclose and claims the following technical solutions:
1.An artificial short interfering peptide of a DAPK1 phosphorylated substrate, characterized by an amino acid sequence as shown in SEQ ID NO . 1, SEQ ID NO .2, SEQ ID NO .3, SEQ ID NO .4 or SEQ ID NO .5 is shown.
2. An artificial short interfering peptide of a DAPK1 phosphorylated substrate, characterized by having an amino acid sequence motif shown as (A) or (B), which is as follows:
   (A)XXX(R/K) (R/K) (R/K) (R/K)X2(S/T/A)X1 XXX;
   (B)XXX(R/K) (R/K) (R/K)X2(R/K) (S/T/A)X1 XXX

Wherein each X can be independently selected from any amino acid or no amino acid.
X1 is a polar amino acid, selected from asparagine (N), cysteine (C), glutamine (Q), serine (S) or threonine (T);
X2 is a non-polar amino acid selected from alanine (A), isoleucine (I), leucine (L), methionine (M) and valine (V);

Where R/K indicates that either arginine (R) or lysine (K) can be used at that position.

Where S/T/A indicates that either serine (S), threonine (T), or alanine (A) can be used at that position.

Preferably, the amino acid sequence of the short interfering peptide differs from at most two amino acids compared to at least one of the amino acid sequences shown in SEQ ID NO.1, SEQ ID NO.2, or SEQ ID NO.3, that is, has at least 85% identity.

Preferably, the amino acid sequence of the short interfering peptide is as shown in any one of SEQ ID NO. 6-54.
3.Artificial short interfering peptides as described in Example 2, wherein the amino acid sequence is represented by any of SEQ ID NO. 6-54.
4.Artificial short interfering mimetic peptides as described in Example 2, wherein the structure is a cyclic peptide or a head-to-tail cyclized peptide, such as cyclization through amide bonds, side chain cyclization, thioester bond cyclization, lactone bond cyclization, cyclization through oxidation between Se-Cys and Se-Cys, or cyclization through disulfide bonds.
5. a reverse peptides of the artificial short interfering peptides described in any one of Examples 1-3, wherein, compared to the artificial short interfering peptides, the amino acid sequence in the peptide chain is reversed from the C-terminus to the N-terminus.
6. A retro-inverted D-peptides of the artificial short interfering peptides described in any one of Examples 1-3. The retro-inverted D-peptides are obtained by replacing each L-amino acid residue in the artificial short interfering peptides with its corresponding D-amino acid residue. The amino acid sequence is reversed, while the original spatial orientation and chirality of the side chains are maintained to be the same as those in the artificial short interfering peptides, thereby preserving a similar side chain topology structure as the artificial short interfering peptides.
7. A derivative peptides of the artificial short interfering peptides described in any one of Examples 1-3, characterized by the substitution of one or more amino acids in the artificial short interfering peptides with their corresponding D-amino acids or beta-homo- amino acids.
8. A polypeptide, characterized by the assembly of two or more short peptides in a parallel manner, wherein the C-terminus of each short peptide is free, and the N-termini of all short peptides are aggregated together for connection with a delivery vector,
   wherein the short peptides are selected from the artificial short interfering peptides described in any one of Examples 1-3, the reverse peptides described in Example 5, the retro-inverted D-peptides described in Example 6, or the derivative peptides described in Example 7.
9.A fusion peptide, characterized by the fusion of one or more delivery vectors at the N-terminus or C-terminus of a short peptide.

The short peptides are selected from the artificial small molecule interfering peptides described in any one of Examples 1-3, the reverse peptides described in Example 5, the retro-inverted D-peptides described in Example 6, or the derivative peptides described in Example 7.

10. According to the polypeptides described in Example 8 or the fusion peptides described in Example 9, wherein the delivery vectors are selected from transmembrane peptides, ligands, protein transduction domains (PTDs), antibodies, or polymeric polymers;
wherein the transmembrane peptides are selected from cationic cell-penetrating peptides, amphipathic cell-penetrating peptides, hydrophobic cell-penetrating peptides, or artificial cell-penetrating peptides;
wherein the polypolymers are selected from polyethylene glycol (PEG), polylactide, poly(lactide-co-glycolide), polyglycolic acid, polycaprolactone, polyethylene oxide, polydioxanone, polypropylene fumarate, trimethylene carbonate, polyurethane epoxy, ester amide, β-hydroxybenzoate, α-hydroxy acids, polyhydroxyalkanoate, polyhydroxybutyrate, polyimide carbonate, polyorthoester, polyanhydride, hyaluronic acid, chitosan, cellulose, gelatin, or collagen.

11.The fusion peptides according to Example 10, the characteristics are as follows:
the cationic cell-penetrating peptides are selected from a group comprising the amino acid sequences shown from Seq. ID No. 55 to Seq. ID No. 72.
the amphipathic cell-penetrating peptides are selected from a group comprising the amino acid sequences shown from Seq. ID No. 73 to Seq. ID No. 81.
the hydrophobic cell-penetrating peptides are selected from a group comprising the amino acid sequences shown from Seq. ID No. 82 to Seq. ID No. 85.
the artificial cell-penetrating peptide has the amino acid sequence shown in Seq. ID No. 86.

12.The fusion peptides according to Example 11, the characteristics are as follows:
The artificial short interfering peptides have amino acid sequences represented by any one of SEQ ID NO. 1-3 and SEQ ID NO. 6-54. The transmembrane peptides have amino acid sequences represented by any one of Seq. ID No. 55-86.

13. The fusion peptides according to Example 10, their characteristics include having amino acid sequences represented by any one of Seq. ID No. 87 to Seq. ID No. 96.

14.An artificially synthesized nucleic acid molecule, characterized by encoding a short peptide selected from the artificial short interfering peptides described in any one of Examples 1-3 and the reverse peptides described in Example 5.

15.An expression vector, characterized by containing the nucleic acid molecule described in 14.

16. An expression system, characterized by being a cellular or cell-free expression system and containing the vector described in Example 15.

17.An expression product, characterized by being expressed by the expression system described in Example 14, and its main component is the short peptide. The short peptide is selected from the artificial short interfering peptides described in any one of Examples 1-3 and the reverse peptides described in Example 5.

18. A kind of medicine, , characterized by containing a peptide molecule and pharmaceutically acceptable impurities, excipients, solvents, protectants, adjuvants, carriers, and/or excipients, wherein the peptide molecule is either:
(1) A artificial short interfering peptide described in any one of Examples 1-3, a reverse peptide described in Example 5, a retro-inverted D-peptide described in Example 6, or a derivative peptide described in Example 7, or
(2) A modified product of a short peptide, where the short peptide is selected from the artificial short interfering peptides described in any one of Examples 1-3, the reverse peptide described in Example 5, the retro-inverted D-peptides described in Example 6, or the derivative peptide described in Example 7, whereinthe modifications include one or more of the following: N-terminal or C-terminal modifications, Labeling, Cyclization, Lipidation, N-methylation

Acetylation or palmitoylation Glycosylation, Biotinylating, PEGylation and Fluorescent labeling.

19.The medicine according to Example 18 is characterized by the following dosage forms: inhalation aerosol, oral formulation, intravenous administration, intra-arterial administration, intracranial administration, intraperitoneal administration, intranasal administration, intramuscular administration, subcutaneous administration, intra-articular or intra-cavity administration, sternal administration, and intraspinal administration formulations.

20.A pharmaceutical use of short peptides is characterized by selecting the short peptides from any one of the artificial short interfering peptides described in Examples 1-3, the reverse peptides described in Example 5, the retro-inverted D-peptide described in Example 6, or the derivative peptides described in Example 7.
wherein the pharmaceutical use refers to the preparation of drugs for the treatment or prevention of diseases associated with excitotoxic amino acid toxicity mechanisms. The diseases include but are not limited to stroke, traumatic brain injury, spinal cord injury, neonatal hypoxic-ischemic encephalopathy, neurodegenerative diseases, depression, and autism.
the neurodegenerative diseases referred to include multiple sclerosis, Alzheimer's disease, amyotrophic lateral sclerosis, Parkinson's disease, or Huntington's disease.

21. A pharmaceutical use of short peptides is characterized by selecting the short peptides from any one of the artificial short interfering peptides described in Examples 1-3, the reverse peptides described in Example 5, the retro-inverted D-peptides described in Example 6, or the derivative peptides described in Example 7;
wherein the pharmaceutical use refers to the preparation of drugs for the treatment or prevention of physiological abnormalities associated with the DAPK1-PKD1 pathway. The diseases include but are not limited to stroke, traumatic brain injury, spinal cord injury, neonatal hypoxic-ischemic encephalopathy, neurodegenerative diseases, depression, and autism;
wherein the neurodegenerative diseases referred to include multiple sclerosis, Alzheimer's disease, amyotrophic lateral sclerosis, Parkinson's disease, or Huntington's disease.

22.A pharmaceutical combination, characterized by comprising the medicine described in embodiment 18 or 19 and an anticoagulant or antiplatelet agent; preferably, the medicine described in embodiment 18 or 19, along with the anticoagulant or antiplatelet drug, is packaged together in units based on single dose, daily dose, or course dose;
wherein the anticoagulant agent refers to Acenocoumarol, Warfarin, Dicoumarol, Ethyl biscoumacetate, Phenindione, Phenprocoumon, Diphenadione, Indandione, Tioclomarol, Bemiparin, Nadroparin, Dalteparin, Enoxaparin, Tinzaparin, Sulodexide, Idraparinux, Danaparoid, Fondaparinux, Idraparinux, Tenecteplase, Desirudin, Apixaban, Dabigatran, Edoxaban, Rivaroxaban, Hirudin, Bivalirudin, Lepirudin, Defibrin polynucleotide, Antithrombin III, heparin, Coumatetralyl, Dabigatran, Apixaban, Enoxaparin, Sulodexide, recombinant tissue plasminogen activator (rtPA), tissue plasminogen activator (tPA), Alteplase, Reteplase, Tenecteplase, Urokinase, Streptokinase, Anistreplase, Monteplase, Ancrod, Fibrinolysin, Brinase, or the combination thereof..

The antiplatelet agent refers to Clopidogrel, Ticagrelor, Prasugrel, Dipyridamole, Cilostazol, Ticlopidine, Eptifibatide, Aspirin, Abciximab, Tirofiban, Beraprost, Prostacyclin, Iloprost, Aloxiprin, Carbasalate calcium, Indobufen, Triflusal, Picotamide, Truquban, Cloricromen, Ditazol, or the combination thereof.

23.The invention also relates to the therapeutic use of short peptides for treating diseases, wherein said short peptides are selected from any one of the artificial short interfering peptides described in embodiments 1-3, the reverse peptides described in embodiment 5, the retro-inverted D-peptides described in embodiment 6, or the derivative peptides described in embodiment 7;
the dosage for administration ranges from 0.001 mg/kg body weight to 50 mg/kg; the concentration of the short peptide described herein can be widely varied, and can be selected based on the chosen mode of administration and the characteristics of the subject, such as body weight, age, gender, etc.

The preferred dosage range is from 0.01 mg/kg body weight to 50 mg/kg body weight.

An further preferred dosage range is from 0.1 mg/kg body weight to 10 mg/kg body weight.

Alternatively, the dosage range can be adjusted to optimize the therapeutic regimen for an individual subject or a group of subjects.

The diseases include but are not limited to stroke, traumatic brain injury, spinal cord injury, neonatal hypoxic-ischemic encephalopathy, neurodegenerative diseases, depression, and autism.

The neurodegenerative diseases refer to Multiple sclerosis, Alzheimer's disease, Amyotrophic lateral sclerosis, Parkinson's disease, or Huntington's disease.

According to reports, DAPK1 is activated and leads to the phosphorylation of Protein Kinase D1 (PKD1), which interacts with DAPK1, in 293T cells and human umbilical vein endothelial cells following oxidative stress damage. The activation of PKD1 is critical for binding to Apoptosis Signal-regulated Kinase 1 (ASK1) and for the phosphorylation of c-N-terminal kinase (JNK), which is induced downstream. Studies have also shown that the ASK1-dependent JNK signaling pathway mediates cysteine-dependent apoptosis and non-cysteine-dependent necrosis in cells after ischemia. However, there have been no reports confirming whether the DAPK1-PKD1 pathway in neurons is involved in the cascade reaction that leads to cell death under ischemia and hypoxia. It is unclear whether interfering with or blocking the DAPK1-PKD1 pathway can effectively reduce ischemic neuronal damage and become a new molecular target for developing drugs to treat ischemic stroke.

The inventors hypothesized the effect of the DAPK1-PKD1 pathway on ischemic neuronal damage and designed a PKD interfering peptide based on this hypothesis. A series of small-molecule peptides with the same motif were derived from this peptide. These peptides were tested in cell and animal models, and the technical solution of the invention was obtained. PKD1 refers to protein kinase D1, a serine/threonine kinase belonging to the protein kinase D (PKD) family that is widely expressed in cells and has unique structural, enzymatic, and regulatory characteristics. The PKD family has three members, PKD1, PKD2, and PKD3. Among them, PKD1 has been studied the most, and it is generally believed that PKD2 and PKD1 are similar in distribution and function, while PKD3 mainly shuttles between the cytoplasm and the nucleus. In terms of structural domains, the three members of PKD have a high degree of homology. PKD has been reported to participate in a variety of cellular functions, including Golgi organization and membrane-guided transport, transfer, immune response, cell apoptosis, and cell proliferation (doi:10.1152/physiol.00037.2010 and Advances in Physiology Education, Vol. 42, No. 5, 2011).

The short peptide drugs developed by the present invention were applied to in vitro oxidative stress injury models and in vivo animal stroke models. The experimental data demonstrate that the short peptide drugs developed by the present invention have significant neuroprotective effects in the glutamate-induced apoptosis model. They can effectively inhibit primary neuronal cell damage caused by oxygen glucose deprivation by interfering with the DAPK1-PKD1 pathway in neurons, inhibiting downstream signals that lead to neuronal cysteine-dependent apoptosis and non-cysteine-dependent necrosis, and reducing brain damage after ischemic stroke. Therefore, the short interference peptides developed by the present invention can be used to prepare therapeutic or prophylactic drugs related to the mechanism of excitatory amino acid toxicity for diseases, including but not limited to stroke, traumatic brain injury, spinal cord injury, neonatal hypoxic-ischemic encephalopathy, neurodegenerative diseases (such as multiple sclerosis, Alzheimer's disease, amyotrophic lateral sclerosis, Parkinson's disease, Huntington's disease), depression, and autism.

### Term Definition

Natural amino acids: The 20 conventional amino acids that can be produced automatically in the body of living organisms, generally L-amino acids.

The term "D-type amino acids" refers to amino acids that are structurally chiral symmetric with L-type amino acids and are synthesized artificially from D-type glycerol. All amino acids, except glycine, have stereoisomers (mirror images of their structures).

The term "Homo-amino acids", also known as long-chain amino acids, is a class of derivatives of natural amino acids. It is the insertion of a methylene group (CH2) into the carbon backbone of a natural amino acid immediately adjacent to the carboxyl group to lengthen the carbon chain; to improve the biological activity and biological stability of peptides.

The terms "peptide", " artificial short peptide", if specifically defined or described, have amino acid sequence characteristics as commonly understood in the art. The term "peptide" or "artificial short peptide", if specifically defined or described, has the characteristics of amino acid sequences as commonly understood in the art, such as the amino acid residues in which are natural L-type amino acids, the amino acid sequence with α-NH2 group at the left end of the amino acid sequence, i.e., the N-terminal on the left, the α-COOH group at the right end and the C-terminal on the right.

The term "reverse peptide" means that the order of amino acids in the peptide chain is changed from the C-terminus to the N-terminus compared to the original peptide chain. For example, the reverse polypeptide of NSGVRRRRLSNVSLC is: NLSVNSLRRRRVGSC.

The term "D-type inverse peptide" means that each L-amino acid residue in the original L-amino acid peptide is replaced by its corresponding D-type amino acid, and its amino acid sequence is reversed while its side chain has the same original spatial orientation and chirality as the original L-amino acid peptide. The amino acid sequence is reversed, and the original spatial orientation and chirality of the side chain is the same as that of the original L-amino acid peptide, i.e., the side chain topology remains similar to that of the original L-amino acid peptide. The side chain topology is similar to that of the original L-amino acid peptide.

### Brief Description of the drawings

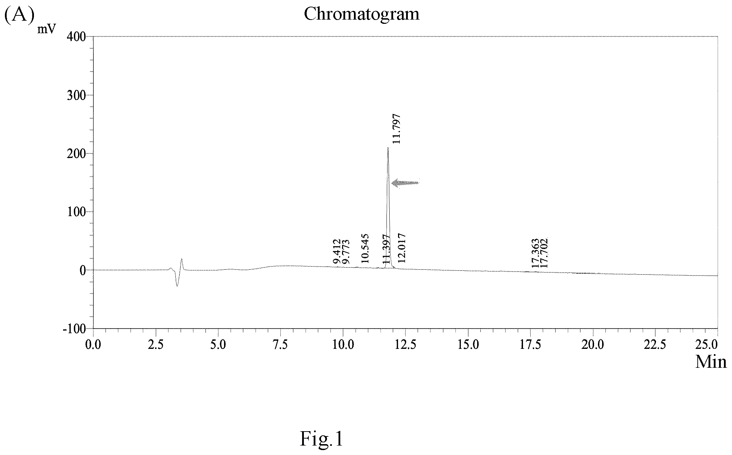

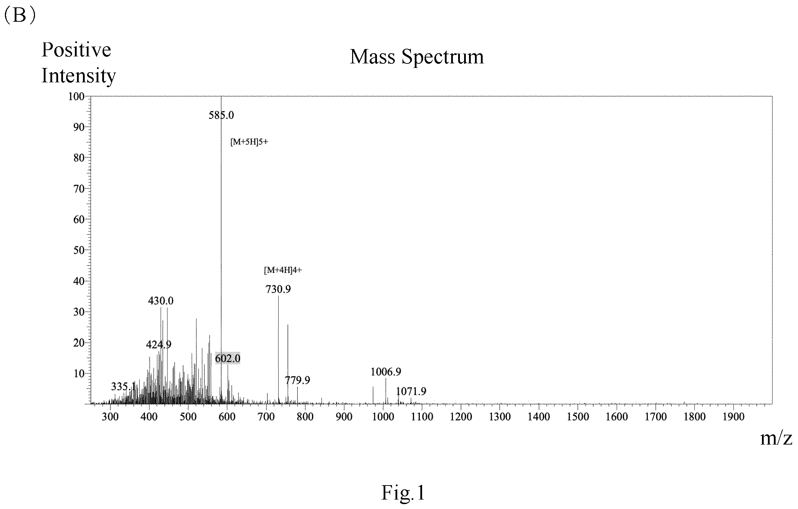

**Figure 1****. The results of the identification and quantitative analysis of the fusion peptide RvTAT-PKD-S205 of the present invention.** (A) RP-HPLC analysis chromatogram of the purified product of RvTAT-PKD-S205: After synthesis and purification to produce RvTAT-PKDS205, it was quantified by reverse HPLC analysis at 210 nm with a peak retention time of 11 .797 min (indicated by arrows) and a final product purity of 98 .3%. (B) Mass spectra of RvTAT-PKD-S205: The peptide RvTAT-PKD-S205 was identified by liquid chromatography mass spectrometry, and the analysis showed that the molecular weight of the synthesized peptide was 2920 .0 Daltons (arrow pointing), and the theoretical molecular weight was 2919 .43 Daltons.

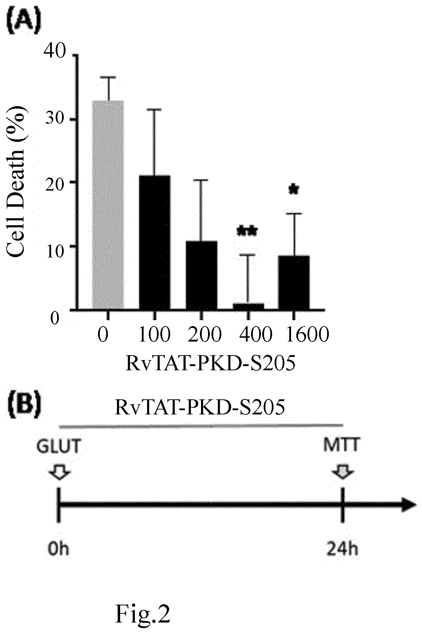

**Figure 2****. Effect of various concentrations of fusion peptide, on glutamate-induced cell death in HT22** cells, wherein(A) HT22 cells containing five different concentrations (0-1600 nM) of RvTAT-PKD-S205 were exposed to 6 mM glutamate (GLUT) for 24 hours at 37°C, then cell cytotoxicity was determined by the MTT assay. The group with 0nM RvTAT-PKD-S205 was as a control. Cell Death rate (%) = 100% x(Control OD - treatment OD) / Control OD. Data were shown as mean ± SEM, (n = 3),
   *p < 0.05, **p< 0.01, One-Way ANOVAfollowed by Multiple Comparisons versus the group with 0nM RvTAT-PKD-S205 (Bonferroni t-test). (B) Timeline and sampling scheme of the experiment.

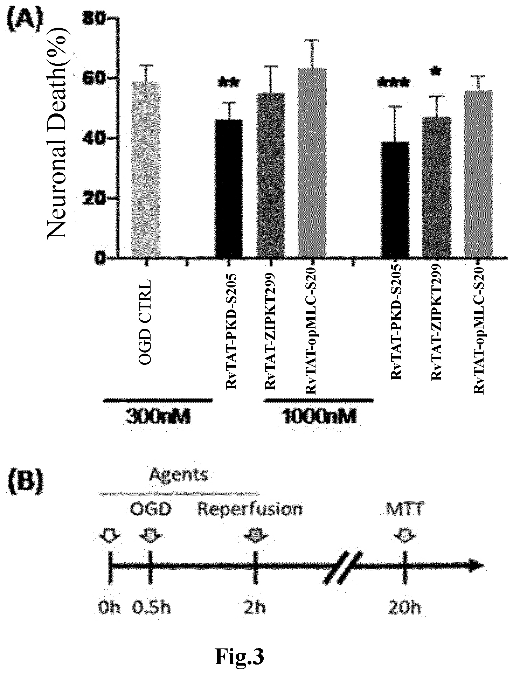

**Figure 3****. The neuroprotective efficacy of the fusion peptide of the present invention in the neuronal against oxygen glucose deprivation/reperfusion (OGD/R) model and the comparison of different fusion peptides, wherein** (A) different concentrations ofRvTAT-PKD-S205, RvTAT-ZIPK-T299, RvTAT-opMLC-S20 were added to rat primary cortical neurons (DIV7) 30 minutes prior to exposure to OGD. To induce OGD, EBSS containing 20mM pH7.2 Sodium dithionite (Na2S2O4) solution with test agents were transiently exposed for 1.5 hrs, afterwards, for reperfusion, OGD culture medium was replaced with normal neurobasal media. The culture cells were further incubated for 20 hours, and cell cytotoxicity was assessed using the MTT assay. Non-OGD treated cell culture was as a control taken as 100% viability, Cell Death rate (%) =100%x (Control OD - treatment OD) / Control OD. Data were shown as mean ± SEM, (n = 3) * p < 0.05, One-Way ANOVA followed by Multiple Comparisons versus OGD CTRL Group (Bonferroni t-test). (B) Timeline and sampling scheme of the experiment.

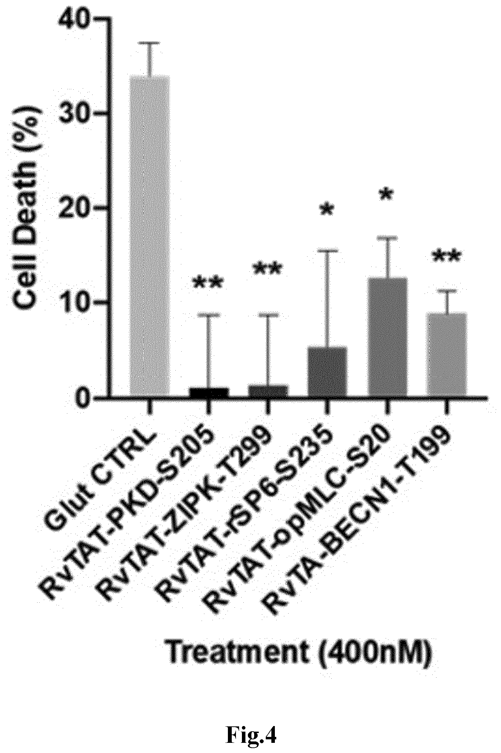

**Figure 4****. Comparison of the efficacy of different fusion peptides on glutamate-induced HT22 cell death,** HT22 cells with the presence of 400 nM variation of the agents of (RvTAT-PKD-S205 RvTAT-ZIPKT299, RvTAT-rSP6-S235, RvTAT-opMLC-S20, RvTAT-BECN1-T199) in culture medium were exposed to 6 mM glutamate for 24 hours at 37°C. After 24 hours following glutamate exposure, cell cytotoxicity was determined by the MTT assay. HT22 cells, without the presence of the test agents in culture medium, were exposed separately to 6 mM Glutamate salt as a control.. Cell Death (%) = 100%x (Control OD - treatment OD) / Control OD. Data were shown as mean ± SEM, (n = 3) * p < 0.05, **p < 0.01, One-Way ANOVA followed by Multiple Comparisons versus Glutamate Control Group (Bonferroni t-test).

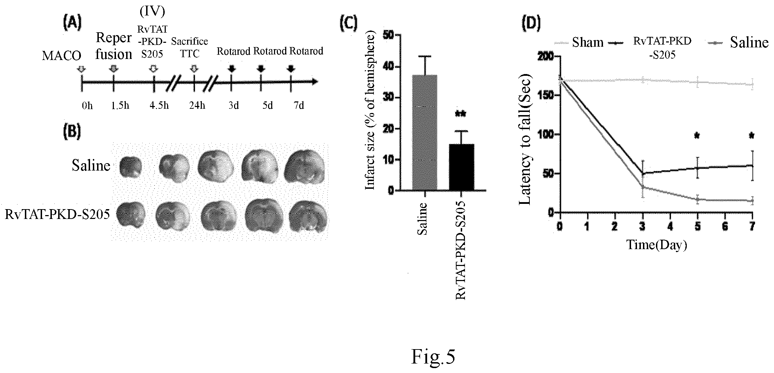

**Figure 5****. The neuroprotective effect of the fusion peptide of the present invention on the transient middle cerebral artery occlusion and reperfusion (MCAO/R) model in Rat Neuroprotective effects, wherein** (A) Timeline and sampling scheme of the experiment. Sprague Dawley rats were subjected to 90 minutes Middle Cerebral Artery occlusion followed by reperfusion (MCAO/R) and treated with 3.5mg/2ml/kg RvTAT-PKD-S20 or some volume Saline via tail vein administered 4.5 hours after ischemia onset. 24 hours afterwards, part of Ischemic reperfusion rats were subjected TTC staining, and the remaining rats were subjected to rotarod test on day 3, 5, and 7. (B) Representative images of brain slices, and (C) Quantification of brain infarct size of rats in Saline group and RvTAT-PKD-S205 treatment group(Saline group, n = 13, RvTAT-PKD-S205 group, n=12). (D) Motor ability as assessed using the rotarod test, the latency time on the Rotarod was recorded. (Sham, n = 8, Saline, n = 6, RvTAT-PKD-S205, n = 10). Data were shown as mean ± SEM, * p < 0.05, **p < 0.01, Student t test. Animals were randomly assigned to treatments groups, , and all procedures were performed by a blind method.

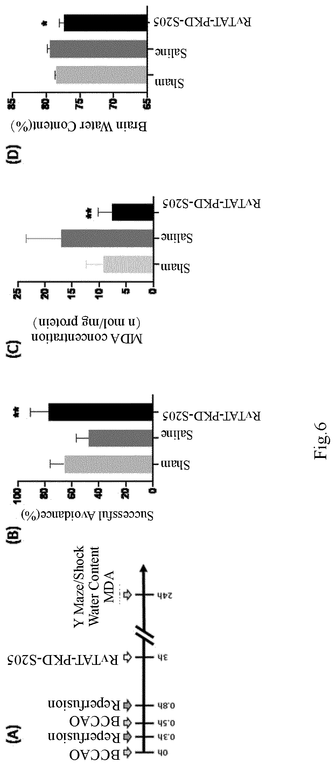

**Figure 6** **The neuroprotective effect of the fusion peptide of the present invention on transient global cerebral ischemia wherein** (A) Timeline and sampling scheme of the experiment. C57BL/6 mice were subjected to two times 20 minutes bilateral common carotid arteries occlusion followed by reperfusion (tBCCAO /R) and treated with 7mg/2ml/kg RvTAT-PKD-S205 or some volume Saline via tail vein administered after 3 hours of the tBCCAO. After 24 hours of reperfusion, the ischemic mice were estimated by the Y maze Passive Avoidance task, as well as the MDA content in hippocampus and brain water content. (B) Learning and memory function of ischemic mice assessed by Y-maze passive avoidance assay in mice that learned to avoid danger arm received a foot-shock. (C) The MDA content in hippocampus collected from Ischemic or sham mice after 24 hours of reperfusion subject to MDA ELISA assay. (D) Measurement of brain water content after 24 hours of reperfusion. Data were shown as mean ± SEM, (n = 11 in each group) , *p<0 .05, **p < 0.01 compared to Saline control group using Student t test. Animal treatments were randomized, and all procedures were performed blinded to treatment.

### Detailed Description of the Embodiments

The embodiments described below by reference to the accompanying drawings are exemplary and are intended to explain the present application and are not to be construed as limiting the scope of the present application. Based on the embodiments disclosed in this application, all other embodiments obtained by those skilled in the art without creative effort are within the scope of protection of this application.

### I. Development of small interfering peptides

The present inventors hypothesized the effect of the DAPK1-PKD1 pathway on ischemic nerve injury by selecting amino acids between positions 197 and 210 of protein kinase D to form a short interfering peptide (PKD-S205) as shown in Seq ID No .1 in Table 1.

Since DAPK1 has multiple phosphorylation-specific substrates in the signaling cascade reaction triggered by the stress response, in addition to interacting with PKD1, DAPK1 also regulatory myosin II light chain (MLC) whose phosphorylation and subsequent activation of myosin-based contractility leads to membrane blebbing. And DAPK1 hosphorylate ribosomal protein S6 (rSP6), thereby reducing translation rates. DAPK1 is also phosphorylates threonine at position 119 of the BH3 structural domain in the autophagic effector protein Beclin 1 (BECN1), promoting the dissociation of Beclin 1 from B-cell lymphoma-extralarge, Bcl-XI,) Inducing autophagy. DAPK1 phosphorylates Zipper-interacting protein kinase (ZIPK) threonine at position 299 influences its intracellular localization and enhances its death-promoting activity.

The present invention also designed interfering peptides targeting the phosphorylation sites of these substrates of DAPK1, such as SEQ ID NO. 2-6 as shown in Table 1.

**Table 1. Artificial Short Interfering peptides designed by the present invention**

| **Serial No.** | **Name** | **SEQ of interfering peptide** | **Description** |
|---|---|---|---|
| SEQ ID NO. 1 | PKD-S205 | SGVRRRRLSNVSL | Interfering peptide of protein kinase D (PKD) |
| SEQ ID NO. 2 | ZIPK-T299 | RKPERRRLKTTRL | Interfering peptide of zipper interacting protein kinase (ZIPK) |
| SEQ ID NO. 3 | rSP6-S235 | QIAKRRRLSSLRA | Interfering peptide of ribosomal protein S6 (rSP6) |
| SEQ ID NO. 4 | opMCL-S20 | KKRPQRRYSNVP | Interfering peptide of the myosin regulatory light chain II |
| SEQ ID NO. 5 | BECN1-T199 | NLSRRLKVTGDL | Interfering peptide of the BH3 domain of Beclin 1 (BECN1) |
| SEQ ID NO. 6 | BECN1-S199 | NLSRRLKVSGDL | Interfering peptide of the BH3 domain S0 mutation of Beclin 1 (BECN1) |
| SEQ ID NO. 7 | BECN1-A199 | NLSRRLKVAGDL | Pseudosubstrate Interfering peptide of the BH3 domain of Beclin 1 (BECN1) |
| SEQ ID NO. 8 | ZIPK-A299 | RKPERRRLKATRL | Pseudosubstrate Interfering peptide of zipper interacting protein kinase (ZIPK) |
| SEQ ID NO. 9 | ZIPK-S299 | RKPERRRLKSTRL | Interfering peptide of S0 mutation zipper interacting protein kinase (ZIPK) |
| SEQ ID NO. 10 | ZIPK-S299-KL | RKPERRRKLSTRL | Interfering peptide of zipper interacting protein kinase (ZIPK) |
| SEQ ID NO. 11 | ZIPK-S299-(-5K) | RKPEKRRKLSTRL | Interfering peptide of zipper interacting protein kinase (ZIPK) |
| SEQ ID NO. 12 | ZIPK-S299-(-4K) | RKPERKRKLSTRL | Interfering peptide of zipper interacting protein kinase (ZIPK) |
| SEQ ID NO. 13 | ZIPK-S299-(-3K) | RKPERRKKLSTRL | Interfering peptide of zipper interacting protein kinase (ZIPK) |
| SEQ ID NO. 14 | ZIPK-S299-(-2R) | RKPERRRRLSTRL | Interfering peptide of zipper interacting protein kinase (ZIPK) |
| SEQ ID NO. 15 | ZIPK-T299(-2A) | RKPERRRAKTTRL | Interfering peptide of zipper interacting protein kinase (ZIPK) |
| SEQ ID NO. 16 | ZIPK-T299(-2I) | RKPERRRIKTTRL | Interfering peptide of zipper interacting protein kinase (ZIPK) |
| SEQ ID NO. 17 | ZIPK-T299(-2M) | RKPERRRMKTTRL | Interfering peptide of zipper interacting protein kinase (ZIPK) |
| SEQ ID NO. 18 | ZIPK-T299(-2V) | RKPERRRVKTTRL | Interfering peptide of zipper interacting protein kinase (ZIPK) |
| SEQ ID NO. 19 | ZIPK-T299(1N) | RKPERRRLKTNRL | Interfering peptide of zipper interacting protein kinase (ZIPK) |
| SEQ ID NO. 20 | ZIPK-T299(1C) | RKPERRRLKTCRL | Interfering peptide of zipper interacting protein kinase (ZIPK) |
| SEQ ID NO. 21 | ZIPK-T299(1Q) | RKPERRRLKTQRL | Interfering peptide of zipper interacting protein kinase (ZIPK) |
| SEQ ID NO. 22 | ZIPK-T299(1S) | RKPERRRLKTSRL | Interfering peptide of zipper interacting protein kinase (ZIPK) |
| SEQ ID NO. 23 | PKD-A205 | SGVRRRRLANVSL | Pseudosubstrate Interfering peptide of Protein Kinase D (PKD) |
| SEQ ID NO. 24 | PKD-T205 | SGVRRRRLTNVSL | Interfering peptide of S0 mutation protein kinase D (PKD) |
| SEQ ID NO. 25 | PKD-S205-LR | SGVRRRLRSNVSL | Interfering peptide of protein kinase D (PKD) |
| SEQ ID NO. 26 | PKD-S205(-5K) | SGVKRRRLSNVSL | Interfering peptide of protein kinase D (PKD) |
| SEQ ID NO. 27 | PKD-S205(-4K) | SGVRKRRLSNVSL | Interfering peptide of protein kinase D (PKD) |
| SEQ ID NO. 28 | PKD-S205(-3K) | SGVRRKRLSNVSL | Interfering peptide of protein kinase D (PKD) |
| SEQ ID NO. 29 | PKD-S205(-2K) | SGVRRRKLSNVSL | Interfering peptide of protein kinase D (PKD) |
| SEQ ID NO. 30 | PKD-S205(-1A) | SGVRRRRASNVSL | Interfering peptide of protein kinase D (PKD) |
| SEQ ID NO. 31 | PKD-S205(-1I) | SGVRRRRISNVSL | Interfering peptide of protein kinase D (PKD) |
| SEQ ID NO. 32 | PKD-S205(-1M) | SGVRRRRMSNVSL | Interfering peptide of protein kinase D (PKD) |
| SEQ ID NO. 33 | PKD-S205(-1V) | SGVRRRRVSNVSL | Interfering peptide of protein kinase D (PKD) |
| SEQ ID NO. 34 | PKD-S205(1C) | SGVRRRRLSCVSL | Interfering peptide of protein kinase D (PKD) |
| SEQ ID NO. 35 | PKD-S205(1Q) | SGVRRRRLSQVSL | Interfering peptide of protein kinase D (PKD) |
| SEQ ID NO. 36 | PKD-S205(1S) | SGVRRRRLSSVSL | Interfering peptide of protein kinase D (PKD) |
| SEQ ID NO. 37 | PKD-S205(1T) | SGVRRRRLSTVSL | Interfering peptide of protein kinase D (PKD) |
| SEQ ID NO. 38 | PKD3-205 | SGVRKRRLSNVSL | Interfering peptide of protein kinase D 3 (PKD3) |
| SEQ ID NO. 39 | PKD2-32 | SGARKRRLSSTSL | Interfering peptide of protein kinase D 2 (PKD2) |
| SEQ ID NO. 40 | rSP6-T235 | QIAKRRRLTSLRA | Interfering peptide of S0 mutation of ribosomal protein S6 (rSP6) |
| SEQ ID NO. 41 | rSP6-S235-LR | QIAKRRLRSSLRA | Interfering peptide of LR mutation ribosomal protein S6 (rSP6) |
| SEQ ID NO. 42 | rSP6-A235 | QIAKRRRLASLRA | Pseudosubstrate Interfering peptide of ribosomal protein S6 (rSP6) |
| SEQ ID NO. 43 | rSP6-S235(-5R) | QIARRRRLTSLRA | Interfering peptide of ribosomal protein S6 (rSP6) |
| SEQ ID NO. 44 | rSP6-S235(-4K) | QIAKKRRLTSLRA | Interfering peptide of ribosomal protein S6 (rSP6) |
| SEQ ID NO. 45 | rSP6-S235(-3K) | QIAKRKRLTSLRA | Interfering peptide of ribosomal protein S6 (rSP6) |
| SEQ ID NO. 46 | rSP6-S235(-2K) | QIAKRRKLTSLRA | Interfering peptide of ribosomal protein S6 (rSP6) |
| SEQ ID NO. 47 | rSP6-S235(-1A) | QIAKRRRASSLRA | Interfering peptide of ribosomal protein S6 (rSP6) |
| SEQ ID NO. 48 | rSP6-S235(-1I) | QIAKRRRISSLRA | Interfering peptide of ribosomal protein S6 (rSP6) |
| SEQ ID NO. 49 | rSP6-S235(-1M) | QIAKRRRMSSLRA | Interfering peptide of ribosomal protein S6 (rSP6) |
| SEQ ID NO. 50 | rSP6-5235(-1V) | QIAKRRRVSSLRA | Interfering peptide of ribosomal protein S6 (rSP6) |
| SEQ ID NO. 51 | rSP6-S235(1N) | QIAKRRRLSNLRA | Interfering peptide of ribosomal protein S6 (rSP6) |
| SEQ ID NO. 52 | rSP6-S235(1C) | QIAKRRRLSCLRA | Interfering peptide of ribosomal protein S6 (rSP6) |
| SEQ ID NO. 53 | rSP6-S235(1Q) | QIAKRRRLSQLRA | Interfering peptide of ribosomal protein S6 (rSP6) |
| SEQ ID NO. 54 | rSP6-5235(1T) | QIAKRRRLSTLRA | Interfering peptide of ribosomal protein S6 (rSP6) |

### II. Cell Penetrating Peptides

Cell penetrating peptides (CPPs) are a class of short peptides that can cross cell membranes or tissue barriers such as the blood-brain barrier independently of specific membrane receptors. CPPs are short peptides that can cross cell membranes or tissue barriers such as the blood-brain barrier independently of specific membrane receptors, and usually do not exceed 30 amino acids. They can transport proteins, RNA, DNA and other macromolecules into cells through endocytosis and direct penetration mechanisms to perform their effector functions, and can be normally decomposed after entering cells, so they have good biocompatibility and low toxicity to cells.

Penetrating Peptides that are well-known in the field can be used in the present invention, as listed in the table below:

**Table 2. Cell Penetrating Peptides**

| Sequence order | Sequence | Title | Description |
|---|---|---|---|
| SEQ ID NO. 55 | GRKKRRQRRRPPQ | TAT (48-60) | Cationic |
| SEQ ID NO. 56 | YGRKKRRQRRR | TAT(47-57) | Cationic |
| SEQ ID NO. 57 | RKKRRQRRR | TAT(49-57) | Cationic |
| SEQ ID NO. 58 | RRRQRRKKRG | RvTAT(47-56) | Cationic |
| SEQ ID NO. 59 | grkkrrqrrrppq | D-TAT | Cationic |
| SEQ ID NO. 60 | KrRrGrKkRrE | cyclic TAT | Cationic |
| SEQ ID NO. 61 | rkkrrrkkrr-Orn-rrr | D-Tat-Om | Cationic |
| SEQ ID NO. 62 | RRRRRRRR | R9 | Cationic |
| SEQ ID NO. 63 | rrrrrrrrr | r9 (D amino acid) | Cationic |
| SEQ ID NO. 64 | KIKKVKKKGRK | dNP2 | Cationic |
| SEQ ID NO. 65 | RRQRRTSKLMKR | PTD-5 | Cationic |
| SEQ ID NO. 66 | RRLSYSRRRF | SynB3 | Cationic |
| SEQ ID NO. 67 | RRIPNRRPRR | HRSV | Cationic |
| SEQ ID NO. 68 | RRRRWWWWRRRR | RWR | chimeric |
| SEQ ID NO. 69 | [WRWRWRWRWR] | [WR]5 | chimeric |
| SEQ ID NO. 70 | RRWWRRWRR-NH2 | RW9 | chimeric |
| SEQ ID NO. 71 | RRLLRRLRR-NH2 | RL9 | chimeric |
| SEQ ID NO. 72 | CHHHHHRRRRRRRRRHHHHHC | HR9 | chimeric |
| SEQ ID NO. 73 | LLIILRRRIRKQAHAHSK | pVEC | Amphipathic |
| SEQ ID NO. 74 | RQIKIWFQNRRMKWKK | pAntp (Penetratin) | Amphipathic |
| SEQ ID NO. 75 | RQIKIWFQ-S5-RRM-S5-WKK | SAH-PEN-2 | Amphipathic |
| SEQ ID NO. 76 | DAATATRGRSAASRPTERPRAPARSASRPRRPV E | VP22 | Amphipathic |
| SEQ ID NO. 77 | KETWWETWWTEWSQPKKKRKV | Pep-1 | Amphipathic |
| SEQ ID NO. 78 | GLWRALWRLLRSLWRLLWRA-cysteamide | CADY | Amphipathic |
| SEQ ID NO. 79 | KQINNWFINQRKRHWK | Kno | Amphipathic |
| SEQ ID NO. 80 | RLLRLLLRLWRRLLRLLR | C6 | Amphipathic |
| SEQ ID NO. 81 | YARAAARQARA | PDT-4 | Amphipathic |
| SEQ ID NO. 82 | GWTLNSAGYLLGKINLKALAALAKKIL | Transportan | Hydrophobic |
| SEQ ID NO. 83 | KLALKLALKALKAALKLA | MAP | Hydrophobic |
| SEQ ID NO. 84 | AGYLLGKINLKALAALAKKIL | TP10 | Hydrophobic |
| SEQ ID NO. 85 | PLILLRLLRGQF | Pept 1 | Hydrophobic |
| SEQ ID NO. 86 | KLALKLALKALKAALKLA | Amphiphilic model peptide | Synthetic |

### III. Fusion peptides

Exemplary embodiments of the present invention select the reverse sequence of TAT(47-57): TAT(RRRQRRKKRG) as the delivery peptide to attach the N-terminal or C-terminus of the small targeting peptide provided by the present invention to form a fusion molecule. The amino acid sequence of some of the fusion peptides of the present invention is listed below.

**Table 3. Sequence information of the fusion peptides provided in the present invention**

| Sequence order | Sequence | Title |
|---|---|---|
| Seq ID No.87 | RRRQRRKKRGSGVRRRRLSNVSL | RvTAT-PKD-S205 |
| Seq ID No.88 | RRRQRRKKRGRKPERRRLKTTRL | RvTAT-ZIPK-T299 |
| Seq ID No.89 | RRRQRRKKRGKKRPQRRYSNVP | RvTAT-opMLC-S20 |
| Seq ID No.90 | RRRQRRKKRGQIAKRRRLSSLRA | RvTAT-rSP6-S235 |
| Seq ID No.91 | RRRQRRKKRGNLSRRLKVTGDL | RvTAT-BECN1-T199 |
| Seq ID No.92 | SGVRRRRLSNVSLRRRQRRKKRG | PKD-S205-RvTAT |
| Seq ID No.93 | RKPERRRLKTTRLRRRQRRKKRG | ZIPK-T299-RvTAT |
| Seq ID No.94 | KKRPQRRYSNVPRRRQRRKKRG | opMLC-S20-RvTAT |
| Seq ID No.95 | QIAKRRRLSSLRARRRQRRKKRG | rSP6-S235-RvTAT |
| Seq ID No.96 | NLSRRLKVTGDLRRRQRRKKRG | BECN1-T199-RvTAT |

### IV.Preparation of fusion peptides

The fusion polypeptide can be expressed using a recombinant expression system and then purified, which is a mature technology in the field and will not be described here.

The fusion polypeptide provided by the present invention is a short peptide, and the use of chemical synthesis method is also ideal.

The fusion peptide used for the subsequent testing of the invention was synthesized by GenScript (China). The peptides were synthesized by solid phase peptide synthesis (SPPS) using Fmoc chemistry.

The process of SPPS includes adding amino acids to the resin one after the other to create the peptide chain. After the synthesis is completed, the Fmoc group is deprotected at the N-terminal, and then the side chain protecting group is deprotected, and then the peptide is cleaved off the resin; The peptide was purified by reversed phase high performance liquid chromatography (RP-HPLC) with acetonitrile plus deionized water and 0.1% trifluoroacetic acid (TFA) as buffer for gradient elution, respectively. The purity was confirmed by analytical RP-HPLC at >95%, and the molecular weight was determined by electrospray ionization mass spectrometry (ESI-MS) to determine the composition (Figure 1). The peptide content was determined by nitrogen determination using Vario MICRO Element Analyzer. The actual peptide amount is calculated by weighing the "purity" content.

The fusion peptides in this patent exist white powder, stored at -20°C protected from light, they can be completely dissolved in water, and are typically prepared as a 200 µM stock solution in either sterile water or saline. The concentration of the peptides used in experiments varies between 0.1 and 2 µM, depending on the specific injury model being studied.

### V.Oxidative stress injury model in vitro

### 1.HT-22 murine hippocampal cell line for Neuroprotection assay

Reagents:
Neurobasal Medium: Thermo Fisher Scientific Gibco 10888022
Dulbecco's Modified Eagle Medium: Thermo Fisher Scientific 10569077
B-27_{TM} Plus Supplement(50X): Thermo Fisher Scientific Gibco A3582801

### 1. 1Glutamate insult test

The cells of HT-22 Mouse Hippocampal neuronal cell line were cultured and maintained in DMEM 10% fetal bovine serum, 100 U/ml penicillin and 100 µg/ml streptomycin. Cells were maintained at a temperature of 37°C in 5% CO2 / 95% air. During agent's treatments and experimentation, HT-22 cells were seeded in 96-well plates by 6 × 10³ cells/well and cultured at 37°C in a CO₂ incubator for 24 h. Afterwards, For neuroprotection assays, HT22 cells were exposure to 6 mM glutamate by removing media and adding Neurobasal Medium with present of 2% B27 (B27 (B-27_{TM} Plus Supplement (50X)) andvarious concentrations of test agents for further 24 hours at 37° Cin the CO₂ incubator. Cell viability was assessed by MTT assay.

### 1.2 Cell Viability Assessment by MTT assay

Cell viability was quantitatively measured by 3-[4,5-dimethylthiazol-2-yl]-2,5-diphenyltetrazolium bromide (MTT) assay.

Water-soluble yellow dye MTT reagent (final concentration 0.5 mg/ml) was added to each well and incubated at 37°C. After 4 hours, the incubate medium was removed and 100 µl of Dimethyl Sulfoxide (DMSO) was added.. After 30 min extraction at room temperature, the absorbance of the formazan solution is determined spectrophotometrically at 570 nm. MTT absorbance data were converted to reflect proportional cell viability relative to both the untreated and treated controls, wherein the untreated control was deemed as 100% viability.

### 1.3 Results

HT22 cells were exposed to 6 mM glutamate and treated with varying concentrations of RvTAT-PKD-S205 peptide. The viability of the cells was assessed using MTT colorimetric assay after 24 hours. The results showed that 100nM, 200nM, 400nM, and 1600nM of RvTAT-PKD-S205 peptide reduced cell death by 11%, 22%, 32%, and 24%, respectively. The concentrations of 400nM and 1600nM showed significant neuroprotective efficacy, but the latter showed a relatively reduced efficacy.

The study results indicate that the fusion peptide RvTAT-PKD-S205 provided by the present invention showed a significant concentration-dependent neuroprotective efficacy in the glutamate induced apoptosis model, and the most effective concentration of the RvTAT-PKD-S205 peptide was 400 nM( as shown in Figure 2).

### 2.Oxygen-Glucose Deprivation (OGD) in primary rat cortical neuronal cultures

Reagents:
Dulbecco's Modified Eagle's Medium (DMEM): Thermo Fisher Scientific 30030
Earle's Balanced Salt Solution (EBSS): Thermo Fisher Scientific 14155063

### 2.1Oxygen-Glucose Deprivation (OGD)

Primary cultures of mixed cortical cell cultures containing both neurons and glia were prepared from embryonic day 15-18 Sprague Dawley rat as described previously [Wenxiang Fan, Xiang Li, Liangliang Huang, Shucheng He, Zhicheng Xie, Yuxin Fu, Weirong Fang, Yunman Li S-oxiracetam ameliorates ischemic stroke induced neuronal apoptosis through up-regulating α7nAChR and PI3K/Akt/GSK3β signal pathway in rats Neurochemistry International Volume 115 May 2018 ,Pages50-60 https://doi .org/10 .1016/j.

Neuint .2018 .01 .008]. Briefly, cerebral cortex was dissociated in Dulbecco's modified Eagle medium (DMEM) and digested at 37°C for 5 min under 0 .25% trypsin digestion, it was stopped by adding fetal bovine serum at a concentration of 10%. This was then obtained by repeated aspiration through a Pasteur pipette after centrifugation at 500 × g for 5 min. Cells were dissociated in Neurobasal medium supplemented with 2% B27 (v/v), 1 mM glutamine, 50 u/ml penicillin and 50 u/ml streptomycin. Cells were plated in 24-well plates (coated with 0 .1 mg/mL poly-D-lysine) at a density of 1.5x 10⁵ cells per well and the medium was subsequently changed every 3 days. Incubation was maintained for 7 days at 37°C in a humidified incubator with 5% CO₂/95% air for use.

Oxygen glucose deprivation were performed according to a previously method [J. Huang, N.D. Kodithuwakku, W. He. Y. Zhou, W. Fan, W. Fang, G. He, Q. Wu, S. Chu, Y. Li The neuroprotective effect of a novel agent N2 on rat cerebral ischemia associated with the activation ofPI3K/Akt signaling pathway Neuropharmacology, 95 (2015), pp. 12-21] with a few modifications to mimic in vivo ischemia and Ischemia/Reperfusion, (I/R) injury, as follows: Prior to exposure to OGD, the primary cultures of the experimental group was treated with different concentrations of the test agent in Neurobasal media for 30 minutes at 37° C in CO incubator, afterwards all medium was removed and the medium of OGD groups were replaced by glucose free EBSS (Earle's balanced salt solution) solution contained Na₂S₂O₄ (20 mM, PH 7.2) with or without agent, while the medium of the control group was replaced by EBSS containing 6mM glucose, then incubated for 1.5 h at 37°C. The OGD challenge was terminated by replacing the medium with normal Neurobasl medium and the cultures were further incubated for a further 20 hours at 37°C. Finally, Cell viability was determined by MTT assay (same as 1.2).

### 2.2 Results

30 min before OGD, the primary neuronal cultures were added with two increasing concentrations of RvTAT-PKD-S205 peptide, followed by the addition of Sodium dithionite to induce hypoxia and glucose deprivation in neurons for 60 min, afterwards, the culture was restored to regular sugar and oxygenated conditions for 20 hours, and then cell viability was measured by MTT assay.

As shown in Figure 3, RvTAT-PKD-S205 had a concentration-dependent quantitative effect relationship on the protective efficacy of neuronal oxygen glucose deprivation system, with 300 and 1000 nM of RvTAT-PKD-S205 significantly reducing neuronal death by 12% and 22% (Figure 3). It is shown that RvTat-PKD-S205 peptide reduces neuronal cell death in a concentration-dependent manner. The peptide at a concentration of 300nM effectively inhibits cellular damage in primary neurons caused by oxygen-glucose deprivation.

### VI. In vivo animal stroke model

### 1.Transient Middle Cerebral Artery Occlusion / Reperfusion (MCAO/R) stoke model in rat

For the acute cerebral ischemia, Middle Cerebral artery Occlusion / reperfusion (MCAO/R) stoke model was established according to previously described methods [Wenxiang Fan, Xiang Li, Liangliang Huang, Shucheng He, ZhichengXie, Yuxin Fu, Weirong Fang, Yunman Li S-oxira cetam ameliorates ischemic stroke induced neuronal apoptosis through up-regulatingα7nAChR and PI3K/Akt/GSK3βsignal pathway in rats Neurochemistry International Volume 115, May 2018, Pages 50-60 https://doi.org/10.1016/j.neuint.2018.01.008] as follow: 220 -250 g male Sprague-Dawley rats were allowed free access to food and water and were housed under constant environmental conditions (a 12/12 h light/dark cycle). Rats were fasted but had free access to water the night before surgery. Rats were anesthetized with chloral hydrate (300 mg/kg, i.p.) and then placed on a heating pad on a surgical table. During the surgical procedure, the body temperature was continuously monitored with a rectal probe and maintained at 36.5~37.0°C. The surgical region was disinfected with povidone-iodine or 70% alcohol. A midline neck incision was made, and the soft tissues over the trachea were gently retracted with a retractor. The common carotid artery (CCA), external carotid artery (ECA), and internal carotid artery (ICA) were carefully isolated from the vagus nerve. Two closely spaced permanent knots were then placed at the distal part of the ECA to prevent the backflow of blood. The pterygopalatine artery was ligated close to its origin with the ICA, then a microvascular clip was placed on the internal carotid artery, and another was placed on the CCA. The ligated ECA was cauterized to create a stump and a 4-0 nylon monofilament with a rounded tip was inserted through the ECA stump into the CCA junction, and a knot was placed below the arteriotomy in the ECA. The microvascular clip placed in the ICA was removed to allow for filament insertion. The filament was carefully inserted up to 18 to 20 mm into the MCA from the CCA junction and stopped when resistance was felt. The monofilament was secured in two places (at the base of the ECA stump and on the ICA) for the remainder of the experiment. After 90 minutes occlusion, the filament was carefully withdrawn until the tip was near the arteriotomy. Following removal of the filament, the knot was tightened in the ECA. The microvascular clip placed in the CCA was removed. When reperfusion was confirmed, the neck skin was sutured. To relieve pain and discomfort in the postoperative period, topical lidocaine gel was applied to the incision region, and the animal received 1.0 mL of normal saline subcutaneously as volume replenishment after the surgery. Sham animals underwent identical procedures without MCAO. At 4.5 h after MCAO, agents were administrated with a volume of 3.5mg/2ml/kg intravenously within 3 min. The rat's body temperature during the recovery time is maintained at 37° C with a heat lamp.

### 2.Brain tissue processing and Infarct volume measurement

24 hours post reperfusion, deeply anesthetized rat was sacrificed and whole brain tissues were removed and coronal sectioned into 2 mm thick slices followed by staining immediately with a 1% 2, 3, 5-triphenyltetrazolium (TTC) solution for 15 minutes at 37°C. The area of the left hemisphere and the unstained area (un-infarcted area) of the right hemisphere were calculated using Image J. (an image processing program developed by the National Institutes of Health) , the percentage of infarction was calculated as follows: Percentage of cerebral infarction area =[ (left hemisphere area - right inferior brain infarct size)/left hemisphere area] x 100%.

Results: As shown in Figure 5(A, B, C), to examine the therapeutic effective treatment of RvTAT-PKD-S205 in-vivo, a post treatment study was conducted with 90 minutes transient MCAO model. SD rats were assigned into 2 groups with either saline alone (control) or saline plus PKD peptide (3.5 mg/kg) treatments by single bolus intravenous injection. RvTAT-PKD-S205 (SEQ ID NO: 1) (n=13) and Saline (n=12) were given 4.5 hours after ischemia onset. Infarct volume (brain injure) was assessed by TTC staining after about 24 hours after MCAO onset. Post treatment with RvTAT-PKD-S205 statistically significantly reduced the volume of total cerebral infarction by approximately 60% as compared with stroke volume in controls after a MCAO stroke (FIG. 5B, C). This result demonstrates that RvTAT-PKD-S205 can act in vivo to reduce ischemic stroke-induced brain injury.

### 3.Assessment of motor functional performance by rotarod

SD rats were tested serially for motor function before and 7 days after focal cerebral ischemia using a Rotarod. Three days prior to MCAO/R surgery, rats were trained on the rotarod for three consecutive days. The initial starting ramp was set at 4 rpm to ensure that animals can be maintained on a rotating pole for 1 minute. After a 10 minutes rest, rats increased steadily up to 20 rpm at 180 seconds and were trained to stay more than 150 seconds on the rod. Animals unable to remain in place on the stationary rod for this duration were repositioned serially until able to do so in the following two days. A trial ended if the animal fell off the rungs or gripped the device and spun around for 2 consecutive revolutions without attempting to walk on the rungs. The duration that rat were able to stay on the rotarod was measured. The mean duration on the device was recorded with 3 rotarod measurements one day before surgery. Rotarod sessions were undertaken on day 3, day 5 and day 7 after MCAO/R (or after sham surgery). For each date of post-ischemic examination, Measurements were taken in triplicate and averaged for each time point. The animal was allowed 15 minutes of rest between each attempt.

Result: To further examine the neurological deficit recovery of TAT-PKD-5205 in-vivo, a post treatment study was conducted with 90 minutes transient MCAO model. Sprague-Dawley rats were assigned into 3 groups: Sham group (No MCAO/R) (n=8), saline group (control) (n=6) or saline plus PKD peptide treatment group (3.5 mg/kg) (n=6). The saline plus PKD peptide treatment group and saline group were give a single dose of TAT-PKD-S205 and saline via tail vein injection 4.5 hours after ischemia onset.Neurological deficit was assessed by Rotarod test at day3, 5 and 7 after MCAO onset.

The motor performance of post treatment with TAT-PKD-S205 rats were significant improved after tMCAO/R. Holding time on rod increase 70% at 5 days and 75% 7 days after tMCAO, respectively, compared to the untreated control group. there were no significant differences in the mean holding time between treated and untreated groups at 3 days after tMCAO (Figure 5D, sham-operated group n = 8, saline group n = 6, RvTATPKD-S205 group n = 10). The results demonstrated that RvTAT-PKD-S205 significantly improved the neurobehavioral induced by ischemic stroke in rats.

### 4.Transient Bilateral Common Carotid Artery Occlusion (tBCCAO) induced cerebral ischemia and agent administration in mice.

Modeling: C57 BL/6 male mice weighing 20-30 g (6-7 weeks old) were allowed free access to food and water and were housed under constant environmental conditions (a 12/12 h light/dark cycle). In short, mice were anesthetized by intraperitoneal injection of 10% chloral hydrate (350 mg/kg) and placed on heating pad to maintain rectal temperature at 37°C and BCCAO surgery was performed according to previously established method. Mouse ventral neck region was shaved. Area was treated with betadine solution then 70% ethanol to cleanse. A small midline skin incision was made in neck. Dissect the medial sternocleidomastoid muscle and isolated carefully from the vagus nerve and connective tissue to expose both common carotid arteries. Non-traumatic vessel clamps were applied to occlude each artery for 20 minutes. The clips were removed, and the blood flow restored for 10 minutes. Then the bilateral common carotid arteries were occlusion again for another 20 minutes and reperfusion for 24 hours. At 3 hours after tBCCAO, agents were administrated with a volume of 7mg/2ml/kg intravenously within 3 min. The mouse's body temperature during the recovery time is maintain at 37° C with a heat lamp. Sham control animals underwent all the surgical procedure except occlusion of BCCAO.

### Experiment: Examine Learning and memory abilities of tBCCAO/R by Passive Avoidance assay

The Y-maze apparatus consisted of three arms (A, B, and C) at 120° angle, connected by a central zone (CZ). The electricity grid (made of stainless steel) was placed underneath each arm; the outer end of each arm had a light bulb providing the light source of the safe zone. Any of the three arms could be set as the starting zone through the computer, which was defined as a non-safe zone after the experiment began. The remaining two arms were randomly divided into a safe zone without foot shock (current stimulation) and a non-safe zone with foot shock by Y-maze video tracking and analysis system.

To examine the therapeutic effect of RvTAT-PKD-S205 peptide on overall cognitive memory deficit improvement in-vivo following stroke, a post treatment study was conducted using a global cerebral ischemia model of transient occlusion and reperfusion of bilateral common carotid arteries and reperfusion model (tBCCAO/R) in mice. In this model, tBCCAO/R primarily caused damage to the hippocampus and impaired the animals' cognitive and memory functions.

The day before surgery, the researchers train the mice to avoid electric shocks caused by light sources, which can minimize the effects of odor. C57BL/6 mice were assigned into 3 groups (n=11 each group): Sham group (No BCCAO), saline group (control) or saline plus RvTAT-PKD-S205 peptide treatment group (7 mg/kg) TAT-PKD-S205 and Saline are given via tail vein injection 3 hours after modeling. Learning and memory skill was quantified by Y maze passive avoidance task 24 hours after tBCCAO/R modeling (Figure 6A).

After 24 hrs BCCAO/R mice were acclimatized to the testing room before conducting the Y-maze and allowed to familiarize themselves with the maze. After 3 min, mice were put in starting area to begin the experiment. A current stimulation of pre-determined intensity (0.05-0.8mA) will be administered. The animal must escape into the adjoining safe zone to avoid receiving the foot shock and keep there for 30 sec. Following the completion of a trial, the animal is replaced in the start location and subsequent trials are completed. The number to escape to the safe zone was recorded; any escapes to non-safe zones were regarded as erroneous. The mean often testing sessions was taken to calculate the average escape percentage. Between animals, the apparatus is completely cleaned with disinfectant and/or alcohol spray.

**Results:** The activity status of each mouse was determined by the percentage of correct avoidance, and observations showed that mice in the RvTAT-PKD-S205 treated group significantly increased the percentage of successful avoidance compared to the saline treated group. RvTAT-PKDS205 peptide improved the decrease in memory index caused by tBCCAO/R, resulting in a 30% increase in memory index in the treated group compared to the untreated group (as shown in Figure 6B). These results suggest that the RvTAT-PKD1-5205 treatment effectively reduces hippocampal neurogenic damage following Globle brain ischemia.

### Mice tBCCAO/R Malondialdehyde (MDA) Assay

MDA is one of the important products of lipid peroxidation and the extent of lipid oxidation was determined by measuring MDA levels. MDA levels in tissues were estimated by the thiobarbituric acid (TBA) method. After the behavior test, the mice were decapitated, and the brain tissues were taken. The hippocampus from brain were homogenized in ice-cold Saline. The supernatants were collected after the lysates were centrifuged at 3,500 r/min for 10 min at 4 °C, and MDA content was determined using MDA TCA kit (Nanjing Jiancheng Bioengineering Institute, Nanjing, China) following the manufacturer's protocol. The results were evaluated from the standard curve and calculated as nM/mg of tissue.

### Assessment of brain edema in tBCCAO/R mice

Mice were assessed for brain edema by the dry/wet weight measurement after 24 hrs BCCAO/R. Wet weight was determined immediately after dissection of the brain, then the tissue was dried at 55°C to a constant weight over 24 hours to determine the dry weight. The percentage of brain water content was calculated as (wet weight - dry weight)/wet weight* 100.

Result: The concentration of MDA in brain tissue was used to assess the severity of neuronal ischemic injury. In comparison to mice treated with saline, the brains of mice treated with RvTAT-PKD-S205 showed a 55% decrease in MDA level, significantly inhibiting the growth of MDA following neuronal ischemic injury (Figure 6C). Brain edema was also evaluated by measuring brain water content, which was reduced by 8.5% after RvTAT-PKD-S205 treatment compared to saline controls (Figure 6D). The results indicate that RvTAT-PKD-S205 attenuates brain edema and oxidative stress injury in the experimental tBCCAO/R model.

**Statistical Analysis:** In the overall animal experiments, a double-blind approach was maintained for the experimental drug group and the control group (sterile saline) during stroke model preparation and assessment of stroke identification and testing parameters. All data are presented as mean ± SEM. Data from more than two groups were analyzed using one-way ANOVA followed by post hoc tests or unpaired two-tailed t-tests with the control group. Multiple comparisons were performed with the control group using post hoc tests or unpaired two-tailed t-tests to assess the difference in means between the treated and untreated control groups. When the data did not meet the ANOVA assumptions described above, multiple comparisons were performed between a nonparametric ANOVA (Kruskal-Wallis ranked one-way analysis of variance) with controls (Dunn method). A value *p<0 .05, **p<0 .01, and ***p<0 .005 were considered statistically significant when comparisons were made.

### VII. Comparison of different fusion peptides

The study involved the synthesis of fusion peptides containing the interfering peptides listed in Table 1. These fusion peptides were tested separately on both the HT22 cell line glutamate attack model and the primary neuronal culture OGD model.

In the OGD model it was found that:
RvTAT-PKD-S205 (SEQ ID NO .1 with the cell penetrating peptides linked at the N-terminal end) provided and its homologous interfering peptides (each one of SEQ ID NO .23 to SEQ ID NO .39 in Table 1 with the cell penetrating peptides attached at the N- or C-terminal) provided significant neuroprotection, reducing cortical neuronal death by 12% and 18% in a dose-dependent manner at 300 and 1000 nM, respectively.

RvTAT-ZIPK-T299 (SEQ ID NO .2 with cell penetrating peptide linked at the N-terminal end) and its homologous interfering peptide (each one of SEQ ID NO .8 to SEQ ID NO .22 in Table 1 with cell penetrating peptide attached at the N-terminal end or C) were also effective in reducing cortical neuronal death by 12% at 1000 nM

RvTAT-opMLC-S20 (SEQ ID NO .4 with the cell penetrating peptide linked at the N-terminal end) peptide did not significantly increase the survival of neurons. With RvTAT-PKD-S205, RvTAT -ZIPK-T299 and RvTAT-opMLC-S20 as examples, the results of comparative neuroprotection experiments are shown in Figure 3.

In the glutamate insult model, it was found that RvTAT-PKD-S205 (N-terminal of SEQ ID NO.1 attached to the cell penetrating peptide) and its homologous interfering peptide (N-terminal or C -terminal of each of SEQ ID NO .23 to SEQ ID NO .39 in Table 1 linked to a cell penetrating peptide) and RvTATZIPK -T299 (N-terminal of SEQ ID NO .2 with cell penetrating peptide linked) and their homologous interfering peptides (N-terminal or C-terminal of each of SEQ ID NO .2 to SEQ ID NO .22 in Table attached to cell penetrating) are the more promising neuroprotective peptides, and both groups of peptides reduce HT22 cell death by an average of about 33%, followed by RvTAT -rSP6-S235 (SEQ ID NO .3 with cell penetrating peptide linked to the N terminus) and its homologous interfering peptide (SEQ ID NO .40 to SEQ ID NO .54 in Table 1 with cell penetrating peptide linked to its N terminus or C terminus), RvTAT-opMLC - S20 (N-terminal of SEQID NO .4 linked to a cell penetrating peptide) and RvTAT-BECN1-T199 (N-terminal of SEQ ID NO .5 linked to a cell penetrating peptide) and their homologous interfering peptides N-terminal or C-linked SEQ ID NO .6 ∼7 linkedcell penetrating peptide) reduced HT22 cell death by an average of about 29%, 25%, and 21% at 400 nM, respectively. With RvTAT-PKD-S205, RvTAT-rSP6-S235, and RvTAT-opMLC-S20 and RvTAT-BECN1-T199 as examples, and the results are shown in Figure 4.

The above results also indicate that although all interfering peptides interact with the phosphorylated functional domain of DAPK1, different DAPK1 substrate motif interfering peptides differed in their neuroprotective efficacy, wherein RvTAT-PKD-S205 and its homologous interfering peptide were the best.

### References

DOI:10 .3969/j.issn .1000-3614 .2019 .02 .001 L Wang, J Liu, Y Yang, B Peng, Y Wang (2019) The Prevention and Treatment of Stroke Still Face Huge Challenges-Brief Report on Stroke Prevention and Treatment in China ,2018. Chinese Circulation Journal ,34:105-19
DOI:10 .3389/fnmol .2016 .00046 P Singh, P Ravanan, P Talwar (2016) Death Associated Protein Kinase 1 (DAPK1): A Regulator of Apoptosis and Autophagy Front. Mol. Neurosci ,9:46
DOI:10 .1007/s 1203 5-016-0008-y S Wang, X Shi, H Li, P Pang, L Pei,H Shen,Y Lu (2017) DAPK1 Signaling Pathways in Stroke: from Mechanisms to Therapies Molecular Neurobiology ,54:4716-4722
DOI:10 .1074/jbc.M505804200 M Shamloo, L Soriano, T Wieloch, K Nikolich, R
Urfer, D Oksenberg (2005) Death-associated protein kinase is activated by dephosphorylation in response to cerebral ischemia J Biol Chem ,280:42290-9
DOI:10 .1038/sj.cdd.4402212 A Eisenberg-Lerer, A Kimchi (2007) DAP kinase regulates JNK signalling by binding and activating protein kinase D under oxidative stress Cell Death & Differentiation ,14:1908-15
DOI:10 .1074/jbc.M414674200 W Zhang, S Zheng, P Storz, W Min (2005) Protein kinase D specifically mediates apoptosis signal-regulating kinase 1-JNK signally induced by H2O2 but not tumor necrosis factor J Biol Chem ,280:19036-44
DOI:10 .1523/JNEUROSCI .4407-08.2008 R Stetler, G Cao, Y Gao, F Zhang, S
Wang, Z Weng, P Vosler, L Zhang, A Signore, S Graham ,J Chen (2008) Hsp27 Protects against Ischemic Brain Injury via Attenuation of a Novel Stress-Response Cascade Upstream of Mitochondrial Cell Death Signalling J Neurosci ,28:13038-55
DOI:10 .1186/1471-2202-4-32 Y Zhang, X Lu, B Bhavnani (2003) Equine estrogens differentially inhibit DNA fragmentation induced by glutamate in neuronal cells by modulation of regulatory proteins involved in programmed cell death. BMC Neurosci ,4:32
DOI:10 .1074/mcp.M700579-MCP200 S Bialik, H Berissi, A Kimchi (2008) A high throughput proteomics screen identifies novel substrates of death associated protein kinase. Mol Cell Proteomics ,7:1089-98
DOI: 10 .1186/s12868-015-0158-2 J Rosario, K Feldmann, T Ahmed, U Amjad, B Ko, J An, T Mahmud, M Salama, S Mei, D Asemota, I Mano (2015) Death Associated Protein Kinase (DAPK) -mediated neurodegenerative mechanisms in nematode excitotoxicity .16:25
DOI:10.1021/bi060413y A Schumacher, A Velentza, D Watterson, J Dresios (2006) Death-Associated Protein Kinase Phosphorylates Mammalian Ribosomal Protein S6 and educes Protein Synthesis. Biochemistry,45:13614-21
DOI:10.1128/MCB.01595-06 A Craig, J Chrystal, K Fraser, N Sphyris, Y Lin,
B Harrion, M Scott, I Dornreiter, T Hupp (2007) The MDMs Ubiquitination Signal in the DNA-Binding Domain of p53 Forms a Docking Site for Calcium Calmodulin Kinase Superfamily Members. MCB,27:3542-55
DOI:10 .1038/embor.2008.246 EZalckvar, H Berissi, L Mizrachy ,Y Idelchuk ,
I Koren, M Eisenstein, H Sabanay, R. Pinkas-Kramaiski, A Kimchi (2009) DAP-kinase mediated phosphorylation on the BH3 domain of beclin 1 promotes dissociation of beclin 1 from Bcl-XL and induction of autophagy. EMBO Rep ,10:285-92
DOI:10 .1128/MCB .24 .19 .8611-8626 .2004 G Shani, L Marash, D Gozuacik, S Bialik, L Teitelbaum,G Shohat, A Kimchi (2004) Death-associated protein kinase phosphorylates ZIP kinase ,forming a unique kinase hierarchy to activate its cell death functions .MCB ,24:8611-26
DOI:10 .1038/sj.cdd.4402212 A Eisenberg-Lerner, A Kimchi (2007) DAP kinase regulates JNK signalling by binding and activating protein kinase D under oxidative stress. Cell Death Differ,14: 1908-15
DOI .org/10 .1021/bi061562j J Fraser, T Hupp (2007) Chemical Genetics Approach to Identify Peptide Ligands that Selectively Stimulate DAPK-1 Kinase Activity. Biochemistry ,46:2655-73
DOI .org/10 .1517/17530050902823829 J Howl, S Jones (2009) Transport molecules using reverse sequence HIV-Tat polypeptides: not just any old Tat? (WO200808225). Expert Opinion on Therapeutic Patents ,19:1329-1333 DOI:10 .1111/j .1747-0285 .2011 .01315 .x Q Guo, G Zhao, F Hao, Y Guan (2012) Effects of the TAT peptide orientation and relative location on the protein transduction efficiency. Chem Biol Drug Des ,79:683-690
DOI: 10.1523/JNEUROSCI .1464-07.2007 H Cui, A Hayashi, H Sun, M Belmares, C Cobey, T Phan, J Schweizer, M Salter, Y Wang, R Tasker, D Garman, J Rabinowiz, P Lu, M Tymianski (2007) PDZ Protein Interactions Underlying NMDA Receptor-Mediated Excitotoxicity and Neuroprotection by PSD-95 Inhibitors. J Neurosci,27:9901-15 DOl: 10 .1016/j.neuint.2018.01.008 W Fan, XLi, L Huang, S He, Z Xie, Y Fu, W Fang, Y Li (2018) S-oxiracetam ameliorates ischemic stroke induced neuronal apoptosis through up-regulatingα7 nAChR and PI3K/Akt/GSK3βsignal pathway in rats. Neurochem Int,115:50-60
DOI: 10 .1016/j.jstrokecerebrovasdis.2019.07.004 D Xu, N Xia, K Hou, F Li,
S Chen, Y Hu, W Fang, Y Li (2019) Clematichinenoside Facilitates Recovery of Neurological and Motor Function in Rats after Cerebral Ischemic Injury through Inhibiting Notch/NF-κB Pathway. J Stroke Cerebrovasc Dis ,28:104288
DOI: 10.1161/01.str.31.1.193 S Ahmed, Y He, ANassief, J Xu, X Xu, C Hsu, F Faraci (2000) Effects of lipopolysaccharide priming on acute ischemic brain injury. Stroke, 31:193-9
DOI: 10.1097/01.WCB.0000096063.84070.C1 I Yonekura, N Kawahara, H
Nakatomi, K Furuya, T Kirino (2004) A model of global cerebral ischemia in C57BL/6 mice. J Cereb Blood Flow Metab,24:151-8
DOI:10 .1155/2018/6381932 M Ru, H Liu (2018) Association between Y-Maze
Acquisition Learning and Major Histocompatibility Complex Class II Polymorphisms in Mice. Biomed Res Int ,2018:6381932
DOI:10 .1074/jbc.M104273200 A Velentza, A Schumacher, C Weiss, M Egli, D
Watterson (2001) A protein kinase associated with apoptosis and tumor Suppression: structure, activity, and discovery of peptide substrates. J Biol Chem ,276:38945-65

## Claims

1. An artificial short interfering peptide for the phosphorylation substrate of DAPK1, **characterized by** its amino acid sequence as shown in SEQ ID NO. 1, SEQ ID NO.2, SEQ ID NO.3, SEQ ID NO.4 or SEQ ID NO. 5 .

2. An artificial small interfering peptide for the phosphorylation substrate of DAPK1, **characterized by** having an amino acid sequence motif shown as (A) or (B), which is as follows:
(A)XXX(R/K) (R/K) (R/K) (R/K) X2(S/T/A) X1 XXX;
(B)XXX(R/K) (R/K) (R/K) X2(R/K) (S/T/A) X1 XXX
Wherein each X can be independently selected from any amino acid or no amino acid.
X1 is a polar amino acid, selected from asparagine (N), cysteine (C), glutamine (Q), serine (S) or threonine (T)
X2 is a non-polar amino acid selected from alanine (A), isoleucine (I), leucine (L), methionine (M) and valine (V);
Wherein R/K indicates that either arginine (R) or lysine (K) can be used at that position;
Wherein S/T/A indicates that either serine (S), threonine (T), or alanine (A) can be used at that position;
Preferably, the amino acid sequence of the artificial short interfering peptide differs from at most two amino acids compared to at least one of the amino acid sequences shown in SEQ ID NO. 1, SEQ ID NO.2, or SEQ ID NO.3, that is, has at least 85% identity.

3. The artificial short interfering peptide according to claim 2, the amino acid sequence is as shown in any one of SEQ ID NO. 6-54.

4. A mimetic peptides of the artificial short interfering peptide according to claim 2, its structure is a stapled peptide or cyclic peptide, such as: head-to-tail cyclized (amide bond), side chain cyclized, thioester bond cyclized, lactone bond cyclized, oxidized Se-Cys to form a ring, or disulfide bond cyclic peptide.

5. The reverse peptide of the artificial small interfering peptide according to any one of claim 1-3 , compared to the artificial small interfering peptide, it has the amino acid sequence of the small interfering peptide reversed from C-terminus to N-terminus.

6. A Retro-Inverted D-Peptide of the artificial small interfering peptide according to any one of claim 1~3, compared to the artificial small interfering peptide, in which each L-amino acid is replaced by corresponding D-amino acid residue, therefore its amino acid sequence is reversed, and side chains' original spatial orientation and chirality remain the same as that in the artificial short interfering peptide, i.e., its side chain topology structure remains similar to that of the short interfering peptide.

7. A derived peptide of the artificial small interfering peptide according to any one of claim 1~3, itis obtained by substituting one or more amino acids of the artificial short interfering peptide with their corresponding D-amino acids or beta-homo-amino acids.

8. A polypeptide, **characterized in that** it is composed of two or more short peptides polymerized in a parallel manner with their C-termini free and N-termini aggregated together for connection with a delivery carrier;
wherein the short peptides are selected from any one of the artificial small interfering peptides of claims 1-3 the reverse peptides of claim, the Retro-Inverted D-Peptide of claim 6, or the derived peptides of claim 7.

9. A fusion polypeptide, **characterized in that** it is composed of one or more delivery carriers fused at the N-terminus or C-terminus of a short peptide;
wherein the short peptides are selected from any one of the artificial small interfering peptides of claims 1-3 the reverse peptides of claim, the Retro-Inverted D-Peptide of claim 6, or the derived peptides of claim 7.

10. The polypeptides according to claim 8 or fusion polypeptide according claim 9, is **characterized in that** the delivery carrier is selected from cell penetrating peptides, ligands, protein transduction domains (PTD), antibodies or polymeric materials;
the cell-penetrating peptides are selected from cationic cell-penetrating peptides, amphipathic cell-penetrating peptides, hydrophobic cell-penetrating peptides, or synthetic cell-penetrating peptides;
the polymeric material is selected from polyethylene glycol (PEG), polylactic acid, poly(lactide-co-glycolide), polyglycolic acid (PGA), polycaprolactone (PCA), polyethylene oxide (PEO), polydioxanone (PDS), polypropylene fumarate, trimethylene carbonate, polyamide epoxy, ester amide, β-hydroxyalkyl methacrylate, α-hydroxy acid, polyhydroxyalkanoate, polyhydroxybutyrate, polyimide carbonate, polyanhydride, polyacid anhydride, hyaluronic acid, chitosan, cellulose, gelatin, or collagen;

11. The fusion polypeptide according to claim 10, is **characterized in that** the cationic cell-penetrating peptides are selected from group of consist of the amino acid sequences shown in Seq. ID No. 55 to Seq. ID No. 72;
the amphipathic cell-penetrating peptides are selected from group of consist of the amino acid sequences shown in Seq. ID No. 73 to Seq. ID No. 81;
the hydrophobic cell-penetrating peptides are selected from group of consist of the amino acid sequences shown in Seq. ID No. 82 to Seq. ID No. 85;
the artificial cell penetrating peptide has the amino acid sequence shown in Seq.ID No.86.

12. The fusion polypeptide according to claim 11, **characterized by** the amino acid sequences of the artificial small interfering peptide as shown in any one of SEQ ID NO. 1-3, SEQ ID NO. 6-54 ; the amino acid sequences of the cell penetrating peptides as shown in any one of SEQ ID NO. 55-Seq.ID NO. 86.

13. The fusion polypeptide according to claim 10, **characterized by** its amino acid sequences as shown in any one of SEQ ID NO. 87-Seq. ID NO. 96.

14. An artificially synthesized nucleic acid molecule, **characterized in that** it is for encoding a short peptide, wherein the short peptide is selected from any one of artificial small interfering peptide in claim 1-3 and reverse peptide of claim 5.

15. An expression vector, **characterized in that** it carries the nucleic acid molecules of claim 14.

16. A expression system, **characterized in that** it is a cell-based or cell-free expression system containing the vector of claim 15

17. An expressed product, **characterized in that** it is expressed by the expression system according to claim 14, and its main component is short peptide; wherein the short peptide is selected from any one of the artificial small interference peptides according to claims 1-3, and the reverse peptide according to claim 5.

18. A kind of medicine comprising a peptide molecule and pharmaceutically acceptable impurities, excipients, solvents, protectants, adjuvants, carriers and/or excipients, wherein the peptide molecule is:
(1) any one of the artificial small interfering peptides of claims 1~3, the reverse peptides of claim 5, the Retro-Inverted D-Peptide of claim 6, or the derived peptides of claim 7; or
(2) a modified product of the short peptide, wherein the short peptide is selected from any one of the artificial small interfering peptides of claims 1~3, the reverse peptides of claim 5, the Retro-Inverted D-Peptide of claim 6, or the derived peptides of claim 7; and the modification includes one or more of the following:
N-terminal or C-terminal modification, labeling, cyclization, lipidation, N-methylation, acetylation, palmitoylation, glycosylation, biotinylating, PEGylation, and fluorescent labeling.

19. The medicine according to claim 18, is characterized byits dosage form selected from:: inhalation aerosol, oral formulation, intravenous administration, intra-arterial administration, intracranial administration, intraperitoneal administration, intranasal administration, intramuscular administration, subcutaneous administration, intra-articular or intra-cavity administration, sternal administration, and intraspinal administration formulations.

20. A pharmaceutically use of short peptides is **characterized by** the short peptide is selected from any one of the artificial small interfering peptides of claims 1-3, the reverse peptide of claim 5, the Retro-Inverted D-Peptide of claim 6, or the derivative peptide of claim 7;
wherein the pharmaceutical use refers to the use of preparation of a medicine for the treatment or prevention of diseases related to the excitatory amino acid toxicity mechanism, the diseases including but not limited to stroke, traumatic brain injury, spinal cord injury, neonatal hypoxic-ischemic encephalopathy, neurodegenerative diseases, depression, and autism;
the neurodegenerative diseases refer to multiple sclerosis, Alzheimer's disease, amyotrophic lateral sclerosis, Parkinson's disease, or Huntington's disease.

21. A pharmaceutical uses of short peptides are **characterized in** the short peptide is selected from any one of the artificial small interfering peptides of claims 1-3, the reverse peptide of claim 5, the retro-inverted D-peptide of claim 6, or the derivative peptide of claim 7; wherein the pharmaceutical use refers to the preparation of drugs for the treatment or prevention of physiological abnormalities related to the DAPK1-PKD1 pathway the physiological abnormalities including but not limited to stroke, traumatic brain injury, spinal cord injury, neonatal hypoxic-ischemic encephalopathy, neurodegenerative diseases, depression, and autism;
wherein the neurodegenerative diseases referred to multiple sclerosis, Alzheimer's disease, amyotrophic lateral sclerosis, Parkinson's disease, or Huntington's disease.

22. A pharmaceutical combination **characterized by** comprising the medicine of claim 18 or 19 and anticoagulant or antiplatelet agent;
preferably, the medicine and anticoagulant or antiplatelet agent are packaged together in units according to single dose, daily dose, or course dose;
wherein the anticoagulant agent refers to Acenocoumarol, Warfarin, Dicoumarol, Ethyl biscoumacetate, Phenindione, Phenprocoumon, Diphenadione, Indandione, Tioclomarol, Bemiparin, Nadroparin, Dalteparin, Enoxaparin, Tinzaparin, Sulodexide, Idraparinux, Danaparoid, Fondaparinux, Idraparinux, Tenecteplase, Desirudin, Apixaban, Dabigatran, Edoxaban, Rivaroxaban, Hirudin, Bivalirudin, Lepirudin, Defibrin polynucleotide, Antithrombin III, heparin, Coumatetralyl, Dabigatran, Apixaban, Enoxaparin, Sulodexide, recombinant tissue plasminogen activator (rtPA), tissue plasminogen activator (tPA), Alteplase, Reteplase, Tenecteplase, Urokinase, Saruplase Streptokinase, Anistreplase, Monteplase, Ancrod, Fibrinolysin, Brinase, or the combination thereof;
wherein the antiplatelet agent refers to to Clopidogrel, Ticagrelor, Prasugrel, Dipyridamole, Cilostazol, Ticlopidine, Eptifibatide, Aspirin, Abciximab, Tirofiban, Beraprost, Prostacyclin, Iloprost, Aloxiprin, Carbasalate calcium, Indobufen, Triflusal, Picotamide, Truquban, Cloricromen, Ditazol, or the combination thereof..

23. A therapeutic use of short peptides are **characterized in** the short peptides is selecting from any one of the artificial small interfering peptides of claims 1-3, reverse peptides of claim 5, retro-inverted D-peptide of claim 6, or derivative peptides of claim 7;
wherein the dosage for administration ranges from 0.001 mg/kg to 50 mg/kg of body weight, wherein the concentration of the short peptides described herein can vary widely and is selected based on the chosen route of administration and factors such as the weight, age, and gender of the subject;
the preferred dosage range is from 0.01 mg/kg to 50 mg/kg;
furthermore, a more preferred dosage range is from 0.1 mg/kg to 10 mg/kg;alternatively, the dosage range may be adjusted to optimize the therapeutic approach for an individual subject or a group of subjects;
wherein the diseases includebut are not limited to, stroke, traumatic brain injury, spinal cord injury, neonatal hypoxic-ischemic encephalopathy, neurodegenerative diseases, depression, and autism;
wherein the neurodegenerative diseases referred to include multiple sclerosis, Alzheimer's disease, amyotrophic lateral sclerosis (ALS), Parkinson's disease, and Huntington's disease.
